# EUROPEAN PATENT SPECIFICATION

(11) **EP 1 140 110 B1**
(45) Date of publication and mention of the grant of the patent: **25.06.2003**
(21) Application number: 99963559.2
(22) Date of filing: 16.12.1999
(51) Int. Cl.: A61K 31/565, A61K 47/48, A61P 5/38

(54) **CLATHRATES OF DEHYDROEPIANDROSTERONE AND CORRESPONDING PHARMACEUTICAL COMPOSITIONS**
EINSCHLUSSKOMPLEXE VON DEHYDROEPIANDROSTERON UND KORRESPONDIERENDE PHARMAZEUTISCHE ZUSAMMENSTELLUNGEN
CLATHRATES DE DEHYDROEPIANDROSTERONE ET COMPOSITIONS PHARMACEUTIQUES CORRESPONDANTES

(30) Priority: 18.12.1998 IT MI982745
(43) Date of publication of application: 10.10.2001
(73) Proprietor: Euphar Group S.R.L., 20124 Milan (IT)
(72) Inventor: CORVI MORA, Paolo, I-29100 Piacenza (IT)
(74) Representative: Gervasi, Gemma, Dr.
(86) International application number: EP9910010
(87) International publication number: WO00037109

(56) References cited:
- WO-A-94/04155
- WO-A-95/15746
- WO-A-97/17992
- US-A- 4 978 532
- US-A- 5 120 720
- PITHA, JOSEF ET AL: "Preparation of drug-hydroxypropyl cyclodextrin complexes by a method using ethanol or aqueous ammonium hydroxide as cosolubilizers" INT. J. PHARM. (1992), 80(2-3), 253-8 CODEN: IJPHDE;ISSN: 0378-5173, 1992, XP002095138
- MCCORMACK, BRENDA ET AL: "Drugs-in-cyclodextrins-in-liposomes: an approach to controlling the fate of water insoluble drugs in vivo" INT. J. PHARM. (1998), 162(1-2), 59-69 CODEN: IJPHDE;ISSN: 0378-5173, 1998, XP002095139
- TAYLOR ET AL.: "BEHAVIOUR AND PHYSIOLOGICAL EFFECTS OF SUPPLEMENTAL EPISODES OF TESTOSTERONE, ITS PRECURSORS AND METABOLITE IN RATS" LIFE SCIENCES, vol. 47, no. 21, 1990, pages 1965-1971, XP002095140
- TAYLOR ET AL.: "Endocrine interactions: adrenal steroids and precursors" AM. J. PHYSIOL., vol. 266, no. 4pt1, 1994, pages E676-E681, XP002095141
- PITHA ET AL.: "Amorphous Water-Soluble Derivatives of Cyclodextrins: Nontoxic Dissolution Enhancing Excipients" J. PHARM. SCI., vol. 74, no. 9, 1985, pages 987-990, XP002095142

## Description

### Prior art

Complexes are known that are obtained by means of processes that enable the inclusion of relatively small molecules in the cavities present in other much bigger molecules, defined as inclusion complexes or clathrates.

In these complexes, the components are not bound by chemical bonds but by the fact that the component of smaller size is prevented from coming out of the said cavities for steric reasons.

The chemical structure of the included molecule is not modified, whilst its chemico-physical characteristics and bio-availability are improved.

On the other hand, it is known how a large amount of research work has been carried out on the clinical use of dehydroepiandrosterone (DHEA), a hormone of the hydrophobic type, which is practically insoluble in water.

The research so far conducted on this hormone has led to conflicting results and hence to diverging opinions as regards its therapeutic use.

In fact, when pharmaceutical forms are tested that do not present good release and constant availability, the risk is to jeopardize the reliability of the experimental result.

In addition, one of the metabolites of the bioconversion of DHEA in the organism is represented by testosterone, and this constitutes a serious disadvantage in that the increase of testosterone blood levels in male subjects imposes the need for particular precautions when adopting a DHEA-based therapy, especially in elderly subjects, on account of the unavoidable recidivation in the pathogenesis of forms of prostatic dysplasia.

To overcome this problem, which considerably limits the possibility of hormonal replacement therapy in aged male subjects, formulations of micronized DHEA have been proposed which seem to be capable of reducing the bioconversion process at the hepatic level (Casson P.R., *et al*. Am. J. Obstet. Gynecol. 174: 649, 1996). Another strategy that has been proposed consists in the preparation of DHEA for transdermal use which enables direct absorption of DHEA in the systemic circulation (WO 94/16709).

However, none of the compositions so far tested has yielded satisfactory results.

### Summary

It has now been found that the problems of the prior art are overcome by means of a clathrate of dehydroepiandrosterone (DHEA) possibly in the form of sulphate, in a matrix of a cyclodextrin characterized by containing a co-solubilization agent selected from the group comprising an amino acid, a polymer such as polyethylene glycol 4000, crospovidone, cellulose, carboxymethyl cellulose and starch, and a bicarboxylic acid such as tartaric, succinic or glutaric acid.

The said clathrate is prepared according to known techniques.

These techniques include:
- mixing of a DHEA solution with a cyclodextrin solution and removal of the solvent by means of spray-drying or lyophilization to obtain the clathrate;
- mixing of a solution of DHEA clathrate with a solution of a co-solubilization agent and recovery of the ternary product so obtained by removal of the solvent;
- co-grinding of the DHEA with a cyclodextrin;
- co-grinding of the DHEA with a cyclodextrin in the presence of vapours of a solvent or of a solvent in liquid form;
- co-grinding of the DHEA clathrate with a co-solubilization agent, possibly in the presence of vapours of a solvent or of a solvent in liquid form.

The clathrate thus obtained present pharmacologically new and suprising characteristics: its administration leads, among other things, to an increased concentration of DHEA in the blood without this entailing any increase in its bioconversion into testosterone, as instead occurs with the administration of DHEA as such.

Moreover, DHEA clathrate leads to an increasing transformation of DHEA to 17-β-estradiol.

Hence, these characteristics enable the use of the said clathrate for the manufacture of a medicament for hormonal replacement therapy in elderly subjects.

### Brief description of the figures

Figure 1 represents the blood levels of DHEA determined by the maximum delta method.
Figure 2 represents the blood levels of DHEA determined by the area-under-the-curve method.
Figure 3 represents the blood levels of DHEA sulphate determined by the maximum delta method.
Figure 4 represents the blood levels of DHEA sulphate determined by the area-under-the-curve method.
Figure 5 represents the molar ratio between DHEA sulphate and DHEA in the blood.
Figure 6 represents the levels of testosterone in the blood.
Figure 7 represents the levels of 17-β-estradiol in the blood.
Figure 8 represents the DSC curve of pure DHEA.
Figure 9 represents the DSC curve of glycine.
Figure 10 represents the DSC curve of α-cyclodextrine.
Figure 11 represents the DSC curve of the physical mixture of DHEA-glycine-α-cyclodextrine.
Figure 12 represents the DSC curve of the clathrate (c-DHEA) obtained by coprecipitation according to Example 11.
Figure 13 represents the DSC curve of the clathrate (GRc-DHEA) obtained by cogrinding according to Example 8.
Figure 14 represents the X-ray spectrum of the pure DHEA.
Figure 15 represents the X-ray spectrum of α-cyclodextrine.
Figure 16 represents the X-ray spectrum of glycine.
Figure 17 represents the X-ray spectrum of the physical mixture of DHEA-glycine-α-cyclodextrine.
Figure 18 represents the X-ray spectrum of the clathrate (c-DHEA) obtained by coprecipitation according to Example 11.
Figure 19 represents the X-ray spectrum of the clathrate (GRc-DHEA) obtained by cogrinding according to Example 8.

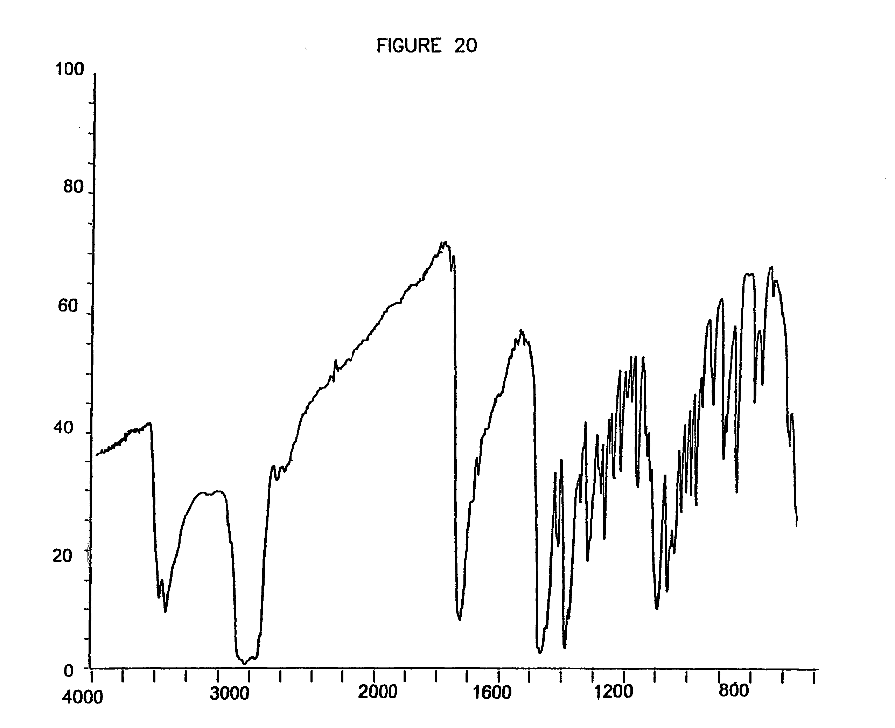

Figure 20 represents the IR spectrum of pure DHEA.

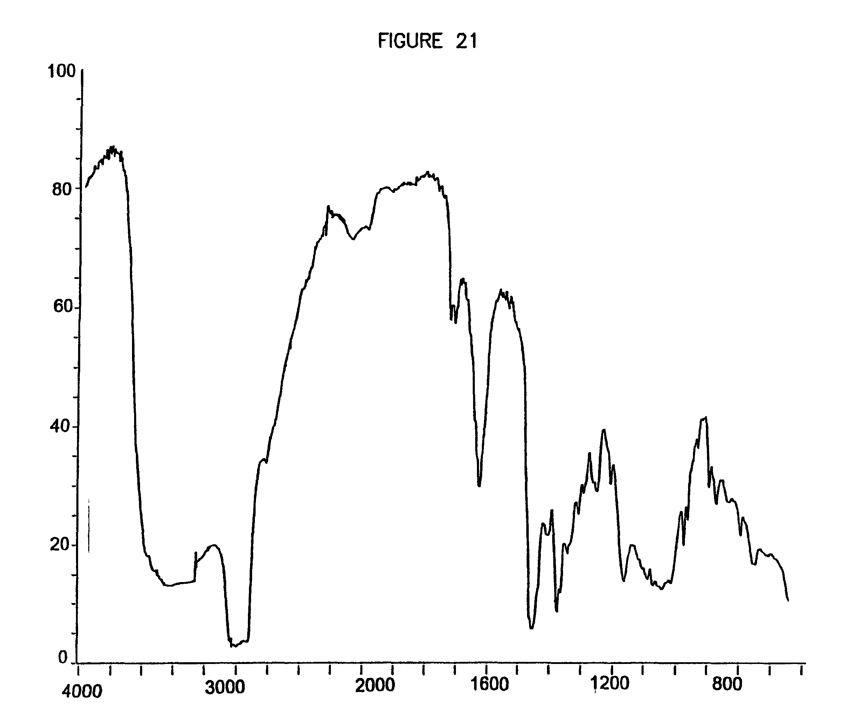

Figure 21 represents the IR spectrum of the α-cyclodextrine.

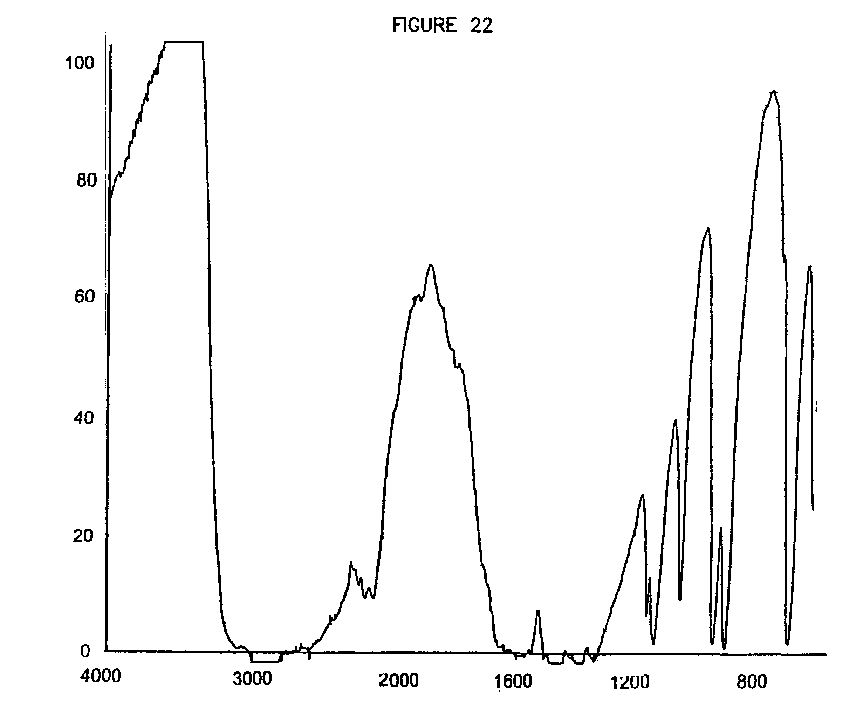

Figure 22 represents the IR spectrum of glycine.
Figure 23 represents the IR spectrum of the physical mixture of DHEA-glycine-α-cyclodextrine.
Figure 24 represents the IR spectrum of the clathrate (c-DHEA) obtained by coprecipitation according to Example 11.
Figure 25 represents the IR spectrum of the clathrate (GRc-DHEA) obtained by cogrinding according to Example 8.

### Detailed description of the invention

The characteristics and advantages of inclusion complexes, or clathrates, of dehydroepiandrosterone (DHEA) or DHEA sulphate according the present invention will be better highlighted in the course of the ensuing detailed description.

The said clathrates comprise DHEA included in a cyclodextrin selected from α-cyclodextrin, β-cyclodextrin and γ-cyclodextrin and a co-solubilization agent selected from the group comprising an amino acid, such as, for example, glycine, lysine, serine and aspartate, a polymer, such as polyethylene glycol 4000, crospovidone, cellulose, carboxymethyl cellulose, starch, and a bicarboxylic acid, such as tartaric acid, succinic acid or glutaric acid.

The said clathrates may be prepared by wet process or dry process, or else by amalgamation (kneading).

In the wet process of preparation, the DHEA and the cyclodextrin are dissolved separately in suitable solvents at a temperature of between 45°C and 50°C.

The solvent preferred for the dissolution of the DHEA is ethanol, whilst the solvent preferred for the dissolution of the cyclodextrin is water.

The cyclodextrin solution having a temperature of 45-50°C is added to the DHEA solution having the same temperature.

The mixture obtained is vacuum-heated at a temperature of between 50°C and 60°C so as to remove the solvents.

This operation is continued until a dry product is obtained (water content according to K.F. < 0,5%).

In the dry process of preparation, the DHEA and the cyclodextrin are co-ground for a sufficient number of hours to obtain the clathrate.

Preferably, the dry process is carried out as follows:

The DHEA and the cyclodextrin are co-ground in a grinding chamber saturated with the vapour of a solvent capable of solubilizing the active substance or of being adsorbed on the surface of the cyclodextrin.

Solvents that are suitable for this purpose are water, methylene chloride, chloroform, methanol, ethanol, and isopropanol.

The grinding mill is of a type suitable for generating high-impact energy. The grinding time ranges from 0.5 to 20 hours.

In the amalgamation or kneading process, the DHEA and the cyclodextrin are kneaded with an organic solvent, and the compound obtained is ground up to complete evaporation of the solvent.

With the processes described above, the DHEA clathrate is obtained in cyclodextrin.

The molar ratio between DHEA and cyclodextrin is preferably 1:1; however, this ratio may be between 3:1 and 1:10.

The clathrate obtained as described above is further treated with a co-solubilization agent to obtain a ternary product by wet process or by dry process, as described previously for the binary product.

The weight percentage of the co-solubilization agent in the ternary product is between 5% and 50%, and is preferably 10%.

The clathrates according to the invention present a high degree of amorphization of the DHEA, a size of the residual crystals in the region of nanometres, a high speed of dissolution and high solubilization kinetics, as well as high bio-availability.

In particular, from pharmacokinetic studies it has emerged that the bio-availability of DHEA clathrate with α-cyclodextrin is approximately twice the bio-availability of DHEA as such.

In addition, the bioconversion of DHEA in testosterone with an administration of 25 mg of DHEA clathrate with α-cyclodextrin comprising glycine as co-solubilization agent, in elderly male subjects, has proved almost insignificant.

The products according to the present invention can thus be used for the preparation of pharmaceutical compositions suitable for the treatment of conditions of hormonal imbalance, especially in elderly subjects.

The said compositions comprise a pharmacologically effective amount of the said products mixed with excipient and diluent substances normally used in pharmaceutical techniques. Preferably, the said compositions contain one portion of the said products formulated so as to enable a fast release of DHEA, and the remaining quantity formulated so as to enable a slow release.

The compositions according to the present invention are used:
- for replacement therapy in all situations of global adrenocortical insufficiency due to primary disease of the gland (Addison's disease) or to primary disease of the diencephalohypophyseal structures (neoplastic, vascular, or surgical pathogenesis) capable of bringing about a secondary hypo-adrenalism;
- for replacement therapy in all situations of partial deficiency of the adrenal cortex, following on prolonged treatment with cortisone for chronic diseases of auto-immune origin;
- for replacement therapy in all the situations, frequently found in the course of ageing, in which the marked reduction of DHEA secretion is accompanied by clinical signs, such as osteopenia, sarcopenia, deficiency in cognitive performance (impaired concentration, attention and memory), mood disorders and immunodeficiency, that together make up a clinical picture definable by the term adrenopause, which may be more in general referred to ageing disorders;
- in women, the treatment may lead to positive effects on conditions having postmenopausal onset, such as vaginal atrophy, libidinal deficiency and urinary incontinence;
- in women with adrenal insufficiency the treatment improves the well-being and sexuality with great improvements in the level of depression and axienty and their physical correlates (e.g. a tendency toward exhaustion);
- in hypogonadal men the treatment may lead to restoration of normal sexual function, prevention of bone loss and muscle wasting and increased ratio of lean body mass to body fat; in these patients additional therapeutic effects can be obtained on cognitive function and non sexual but male-related behaviour;
- in both sexes, therapeutic efficacy is obtained in the treatment of glucide metabolism and lipid metabolism disorders. It follows that DHEA may offer prophylactic and therapeutic effectiveness in regard to obesity and above all in regard to arteriosclerotic diseases.

On the basis of the *in vitro* experimental data and the epidemiological findings, it is also possible to hypothesize a capacity of DHEA to have a favourable action on the development of breast cancer and gastric carcinoma.

Finally, another documented biological effect is represented by the capacity to activate the secretion of the lachrymal gland, and hence to perform a therapeutic role in xerophthalmia.

The therapy envisages the oral administration of a dose of from 12.5 mg to 50 mg/day of DHEA in the form of DHEA clathrate.

To provide an illustration, the following examples of preparation of the compounds according to the present invention and the corresponding pharmacological experimentation are given.

### Example 1

Into a glass reactor equipped with a reflux cooler and protected from the light were introduced 6 g (0.0208 mol) of DHEA and 200 ml of 95% ethanol, and the mixture obtained was heated to 47°C up to complete dissolution of the DHEA.

Separately a solution was prepared of 10 g (0.0102 mol) of α-cyclodextrin in 200 ml of distilled water, and the solution was heated to 47°C.

The α-cyclodextrin solution at a temperature of 47°C was added, under stirring, to the DHEA solution having the same temperature.

This operation was carried out slowly, to prevent the solution from becoming turbid.

A solution was obtained which was practically clear or slightly opalescent.

This solution was transferred into a vacuum reactor pre-heated to a temperature of 50-60°C and was kept at this temperature in vacuum conditions until a dry product was obtained (residual H₂O content according to K.F. < 0.5%).

The dry product obtained was sieved using a 60-mesh sieve, away from the light.

In this way, 14.8 g of a clathrate having a molar ratio between DHEA and α-cyclodextrin of 2:1 were obtained.

On the product obtained, solubility tests and differential scanning calorimetry (DSC) analyses were carried out.

The solubility tests in this example and in the following ones were carried out by dispersing a quantity of product corresponding to 60 mg of DHEA in 100 ml of bidistilled water at 25°C under stirring.

Determination of solubility was carried out after the product had remained in water for 24 hours. Solubility was found to be 11.5 mg of DHEA in 100 ml of water.

At DSC analysis, the melting temperature was found to be 139.8°C and 149.3°C respectively, and the melting heat 158.09 J/g and 10.19 J/g respectively, thus proving the existence of one part of DHEA in the form of clathrate and one residual crystalline part of DHEA.

By comparison, DHEA as such presented a melting temperature of 150.9 and a melting heat of 84.2 J/g.

### Example 2

Example 1 was repeated, with the difference that 3 g (0.0104 mol) of DHEA and 10 g (0,0102 mol) of α-cyclodextrin were used.

A clathrate having a molar ratio between DHEA and α-cyclodextrin of 1:1 was obtained.

Solubility of the product in water was found to be 11.3 mg of DHEA in 100 ml of water.

The melting temperature was found to be 135.2°C and 147.5°C respectively, and the melting heat 130.18 J/g and 55.56 J/g respectively.

### Example 3

Example 1 was repeated, with the difference that 1.5 g (0.005 mol) of DHEA and 10 g (0.0102 mol) of α-cyclodextrin were used.

A clathrate was obtained with a molar ratio between DHEA and α-cyclodextrin of 1:2.

Solubility of the product in water was found to be 11.7 mg of DHEA in 100 ml of water.

The melting temperature was found to be 139.0°C and 150.9°C respectively, and the melting heat 30.42 J/g and 11.06 J/g, respectively.

### Example 4

A mixture consisting of 3 g of DHEA and 10 g of α-cyclodextrin having a grain size of less than 60 mesh was put into the grinding chamber of a high-energy colloidal mill.

The grinding chamber was saturated with methylene chloride vapour at room temperature, and the mixture was ground for one hour.

The product was then vacuum treated to remove the adsorbed methylene chloride, and was finally sifted using a 60-mesh sieve.

The product obtained, consisting of a clathrate with a molar ratio between DHEA and α-cyclodextrin of 1:1, had a solubility in water of 13.3 mg of DHEA in 100 ml of water. The melting temperature was 149.4°C and the melting heat was 13.62 J/g, thus proving that all the DHEA was in the form of clathrate.

### Examples 5 and 6

Example 1 was repeated, with the difference that, instead of α-cyclodextrin, β-cyclodextrin (Example 5) and γ-cyclodextrin (Example 6) were used.

Clathrates were obtained having poorer characteristics as compared to the clathrate of Example 1.

### Example 7

In a mechanical mortar were introduced 0.3 g of DHEA and 1.0 g of α-cyclodextrin, and then 1 ml of ethanol was added.

The mixture was ground vigorously until complete evaporation of the methanol.

A clathrate was obtained having a molar ratio of between DHEA and α-cyclodextrin of 1:1.

Solubility in water was 11.6 mg of DHEA in 100 ml of water.

The melting temperature was 149.9°C and the melting heat was 49.03 J/g.

### Example 8

Example 4 was repeated, with the only difference that in the grinding chamber were introduced 10.0 g of the product obtained from Example 4 in a mixture with 1.25 g of glycine hydrochloride.

A clathrate was obtained containing glycine as co-solubilization agent.

Solubility in water was found to be 12.5 mg of DHEA in 100 ml of water.

The DCS curve is characterised by a wide endothermic band from 60 to 160°C demonstrating the amorphization of the product.

### Example 9

Example 4 was repeated, with the only difference that in the grinding chamber were put 10.0 g of the product obtained from Example 4 and 1.0 of PEG 4000.

The product obtained had a solubility in water of 11.0 mg of DHEA in 100 ml of water.

The melting temperature was found to be 149.8°C, and the melting heat 6.61 J/g.

### Example 10

Example 7 was repeated, with the only difference that 1.0 g of the product obtained in Example 7 was put into the mortar mixed with 0.1 g of glycine hydrochloride.

The product obtained had a solubility in water of 11.8 mg of DHEA in 100 ml of water.

The melting temperature was found to be 149.6°C, and the melting heat was found to be 32.86 J/g.

### Example 11

Example 1 was repeated, with the difference that 3 g (0,0104 mol) of DHEA and 10 g (0,0102 mol) of α-cyclodextrin were used and that 1,6 g of glycine hydrochloride was added in preparing the solution of DHEA.

A clathrate was obtained containing glycine as co-solubilization agent.

The product obtained had a solubility in water of 11,7 mg of DHEA in 100 ml of water.

The melting temperature was found to be 140,2°C and 149,6°C respectively and the melting heat 19,9 J/g and 26,9 J/g respectively.

### Example 12

Example 4 was repeated with the difference that 3 g (0,0104 mol) of DHEA and 20 g (0,0204 mol) of α-cyclodextrin were used.

10 g of the product obtained were introduced in the grinding chamber with 1,0 g of glycine hydrochloride.

A clathrate having a molar ratio between DHEA and α-cyclodextrin of 1:2 containing glycine as co-solubilization agent was obtained.

Solubility of said clathrate in water was found to be 18,7 mg of DHEA in 100 ml of water.

The DCS curve is characterised by a broad, regular endothermic band from 60 to 160°C showing the almost total amorphisation of the system.

### Physico-chemical characterisation of the clathrate DHEA-α-cyclodextrin-glycine

Differential scanning calorimetry analysis (DSC), X-ray diffractometry and infrared spectroscopy were carried out on the following samples:
- Pure dehydroepiandrosterone (DHEA)
- Glycine (Gly)
- α-Cyclodextrin (α-Cd)
- a physical mixture (P.M.) of the three components, in the same ratios present in the clathrates (DHEA: α-Cd in an equimolar ratio, in the presence of 12.5% w/w of glycine)
- clathrate DHEA-α-Cd-glycine (c-DHEA), obtained by precipitation according to Example 11
- clathrate DHEA-α-Cd-glycine (GRc-DHEA), obtained by cogrinding according to Example 8

### A) DSC ANALYSIS

A Mettler TA 4000 appliance with a DSC 25 cell was used.

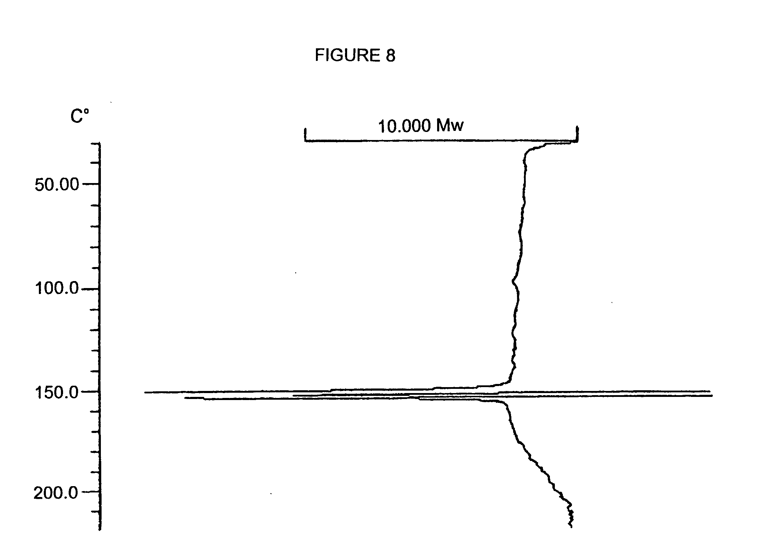

The DSC curve of pure DHEA (Figure 8 -DHEA) presents a thermal profile typical of a pure, crystalline anhydrous substance, characterised by the presence of a single endothermic peak, situated at 150.9°C, with an enthalpy variation of 84.2 J/g, which may be attributed to its melting. At a higher temperature, the onset of an exothermic effect is noted, which may be attributed to a phenomenon of degradation.

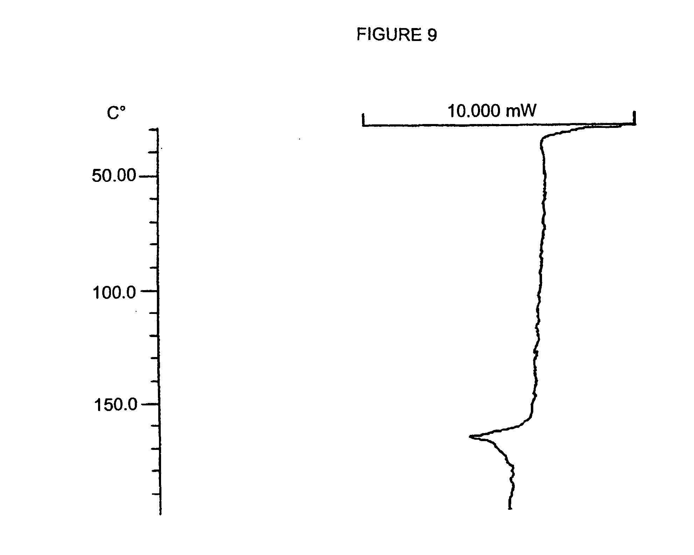

The DSC curve of the sample of glycine (Figure 9 - Gly) presents a single endothermic phenomenon, which may be attributed to its melting, with a peak temperature of 166.3°C, accompanied by an enthalpy variation of 11.6 J/g.

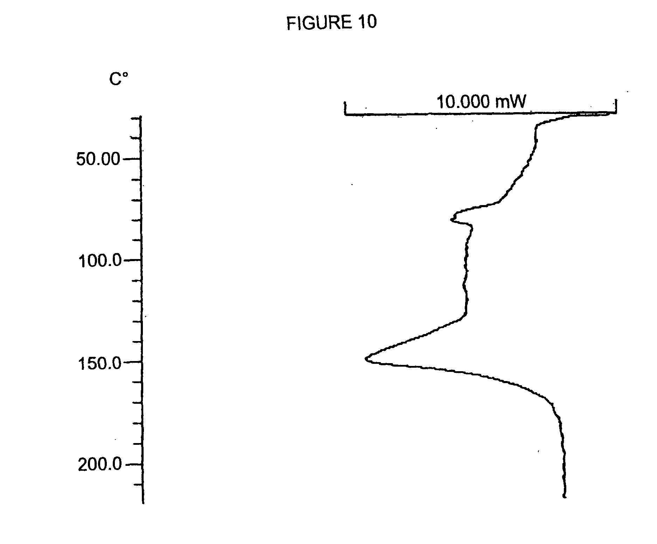

The DSC curve of α-Cyclodextrin (Figure 10 α-Cd) in the temperature range considered, is characterised by a broad endothermic band, with a more intense peak around 150°C, which may be attributed to its dehydration process α-C contains about 10% of crystallisation water).

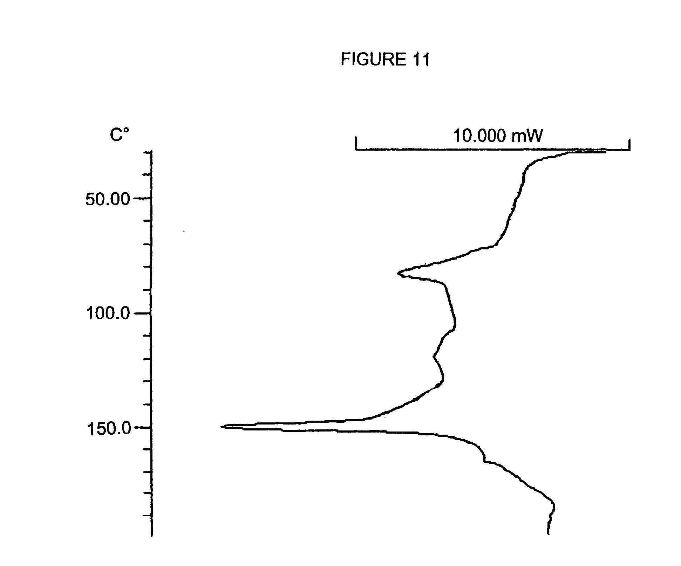

The DSC curve of the physical mixture of DHEA-α-Cyclodextrine-Glycine (Figure 11 - P.M.) corresponds to the overlapping of the thermal curves of its three components, indicating that there has been no interaction between the components themselves. The broad endothermic band of dehydration characteristic of α-Cyclodextrin is clearly recognisable, as well as the distinct endothermic peak of melting of DHEA (Tₚₑₐₖ = 149.5°C), followed by a shoulder of about 165°C, which may be attributed to the melting of the glycine. The enthalpy value obtained by integrating the melting peak of DHEA (ΔH = 213.3 J/g) is clearly increased, due to the partial overlapping of the cyclodextrin dehydration phenomenon.

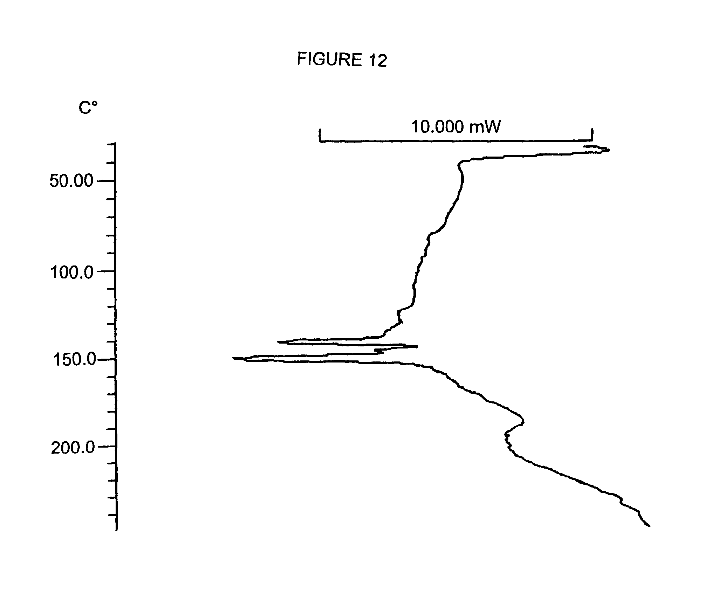

The DSC curve of the clathrate obtained by precipitation according to Example 11 (Figure 12-c-DHEA), is clearly different from that of the corresponding physical mixture (Figure 11- P.M.), which indicates that interaction has occurred. The curve is characterised by the presence of two distinct endothermic peaks, at respectively 140.2°C (ΔH = 19.9 J/g) and 149.6°C (ΔH = 26.9 J/g). The peak at the higher temperature is certainly to be attributed to the melting of the DHEA which did not interact in the formation of the clathrate; in fact its temperature corresponds exactly to the one observed for the DHEA in the physical mixture (Tₚₑₐₖ = 149.5°C), which in turn corresponds to that of pure DHEA (Tₚₑₐₖ = 150.9°C). Instead, the first peak could be attributed to the melting of a new product formed by interaction between the components. The low enthalpy values of both endothermic phenomena indicate a considerable reduction in crystallinity of the system in comparison with the simple physical mixture.

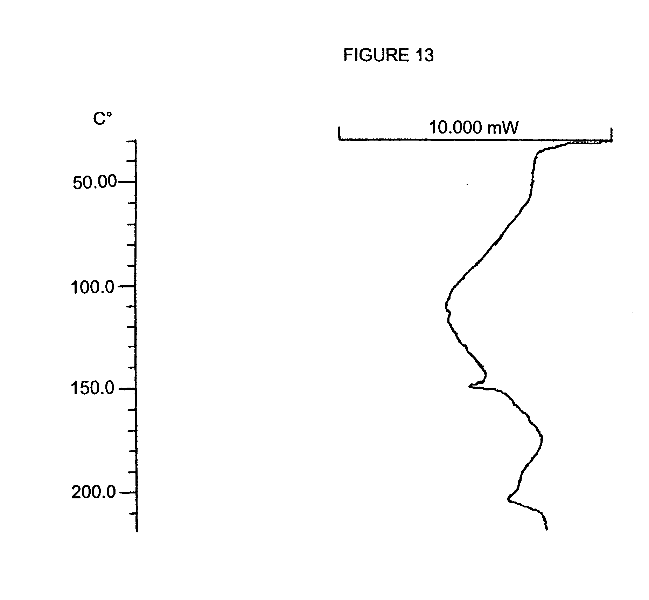

The DSC curve of the clathrate obtained by cogrinding according to Example 8 (Figure 13 - GRc-DHEA) is distinctly different both from that of the physical mixture and from that of the clathrate obtained by precipitation (c-DHEA), and it is characterised by a broad, regular endothermic band from 60 to 160°C. The absence of any other peak indicates that the interaction between the three components, after cogrinding, led to the almost total amorphisation of the system.

It must be stressed that reaching an amorphous state is extremely advantageous, since a solid product in the amorphous state always presents better dissolving characteristics than the corresponding crystalline product.

### B) X RAY ANALYSIS

The X-ray diffraction patterns of the various samples were obtained by means of a Philips PW 130 diffractometer (using Cu Ka radiation), with a scanning speed of 1°/min in the range 10-50° 2θ

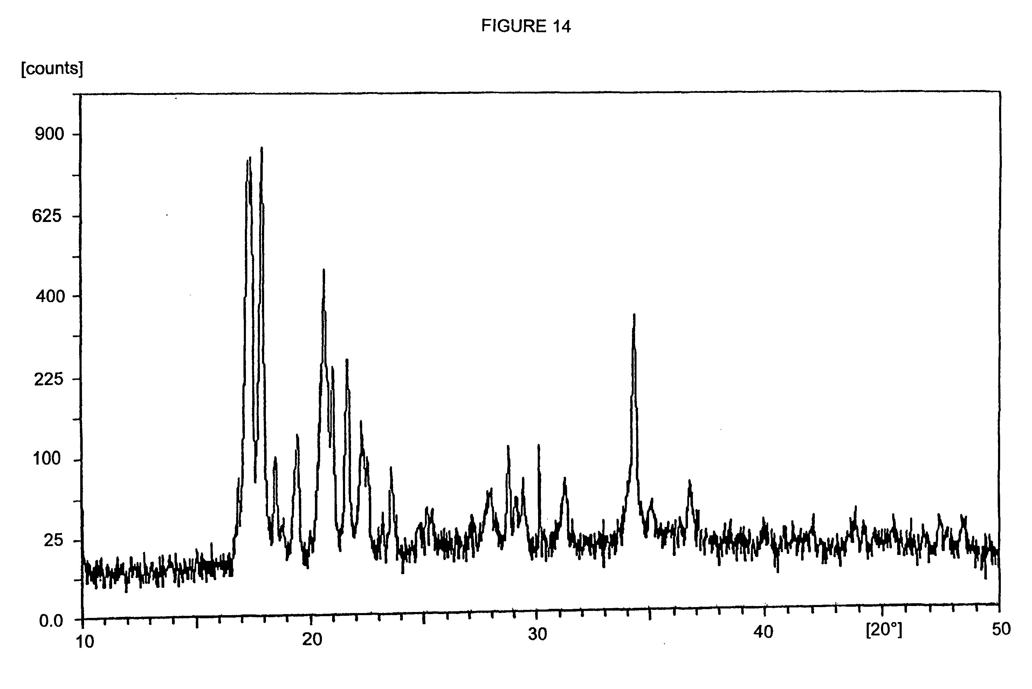

The X-ray spectrum of pure DHEA (Figure 14 - DHEA) is typical of a crystalline product, with a flat base line and numerous peaks of crystallinity, including particularly intense ones at 17.3; 18.1; 20.6; 21.5; 22.3: 34.5° 2θ.

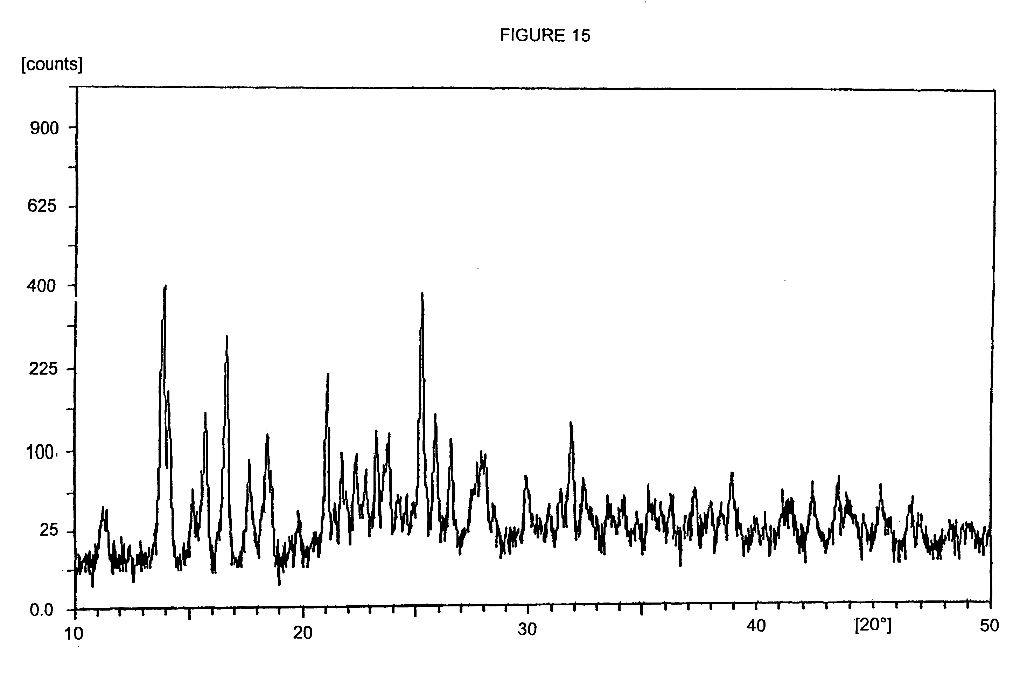

The X-ray spectrum of α-Cyclodextrin (Figure 15 - α-Cd) is also typical of a crystalline product; numerous peaks of crystallinity are observed, including particularly intense ones at 13.8; 16.6; 21; 25.2° 2θ.

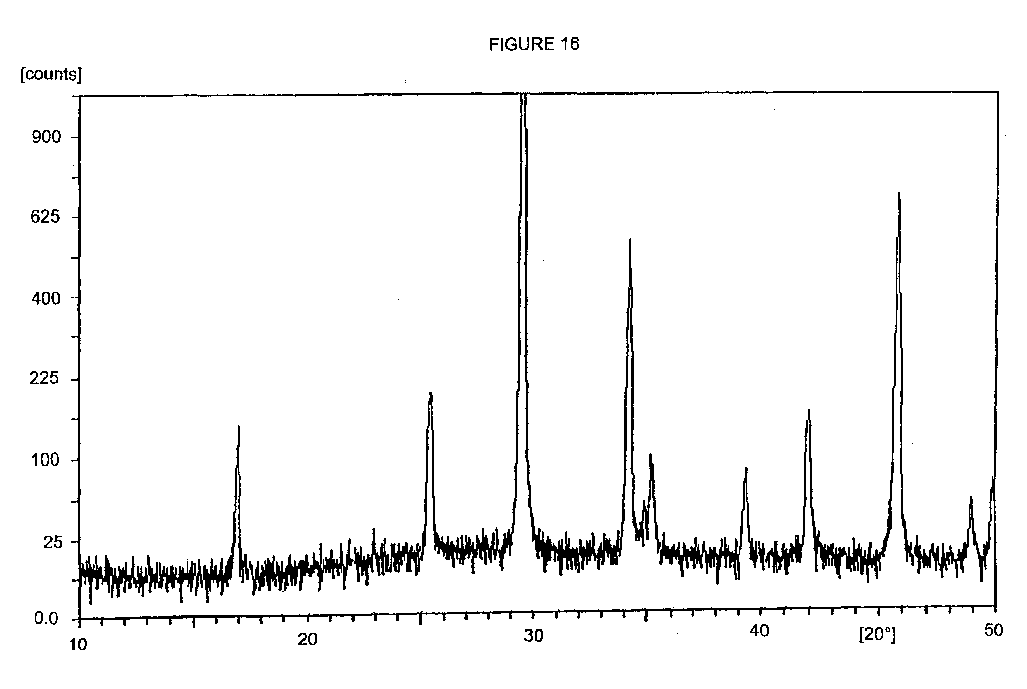

The X-ray spectrum of glycine (Figure 16 - Gly) is also typical of a crystalline product; intense peaks of crystallinity are observed at 16.8; 25.5; 29.5; 34.2; 35.3; 39.5; 42;46° 2θ.

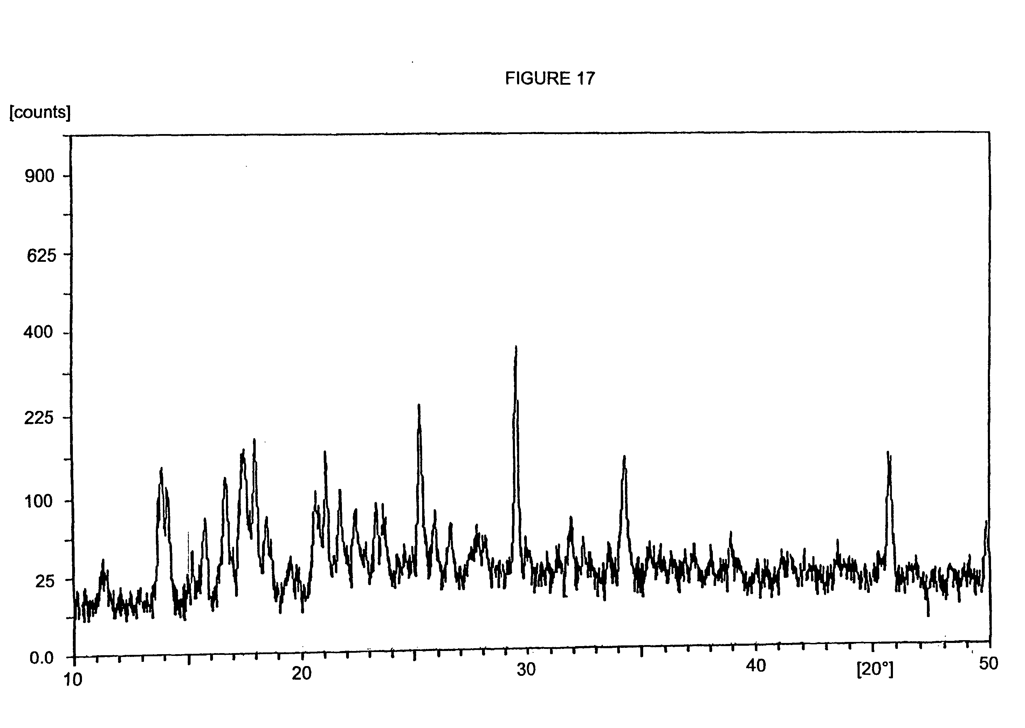

The diffraction pattern of the physical mixture of DHEA-α-Cyclodextrin-Glycine (Figure 17 - P.M.) represents the overlapping of the spectra of the three pure components; numerous peaks of crystallinity may be recognised, with angle 2θ values similar to those observed for the three components, without shifts or evident alterations.

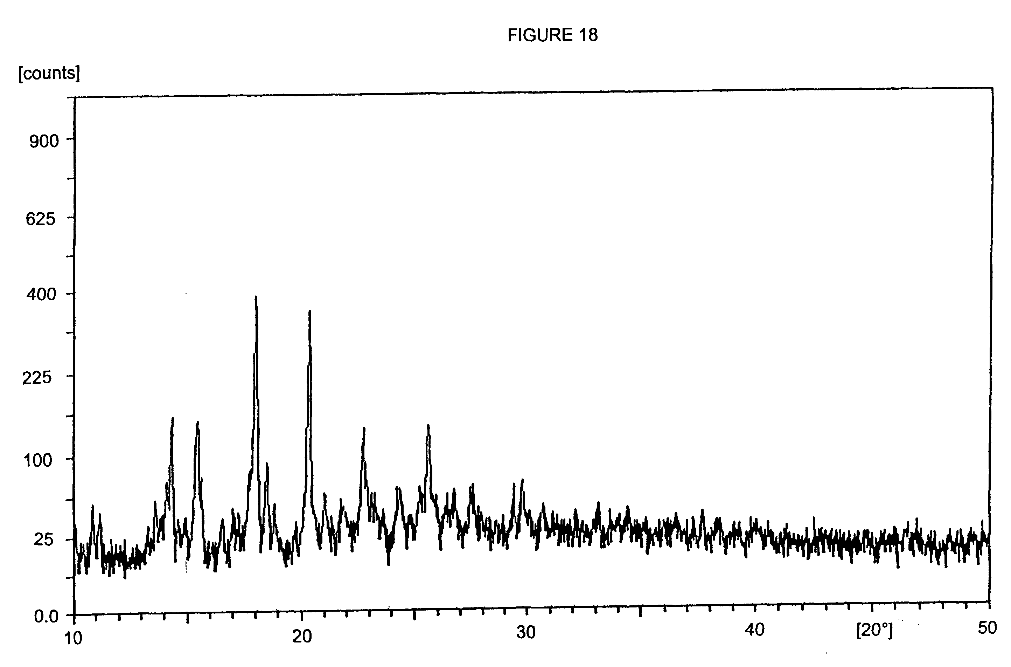

The diffraction pattern of the clathrate obtained by precipitation according to Example 11 (Figure 18 - c-DHEA), is clearly different from that of the corresponding physical mixture. A reduction is observed both in the intensity and in the number of peaks, indicating a reduction of crystallinity of the sample. Also from the comparison with the physical mixture, a variation of the relative intensity of some peaks may be observed, with increased relative intensity of the peaks at 18 and 20.5° 2θ in particular; this phenomenon, accompanied by the shifting of some peaks, would confirm the formation of a new solid crystalline phase, as shown also by the DSC analysis.

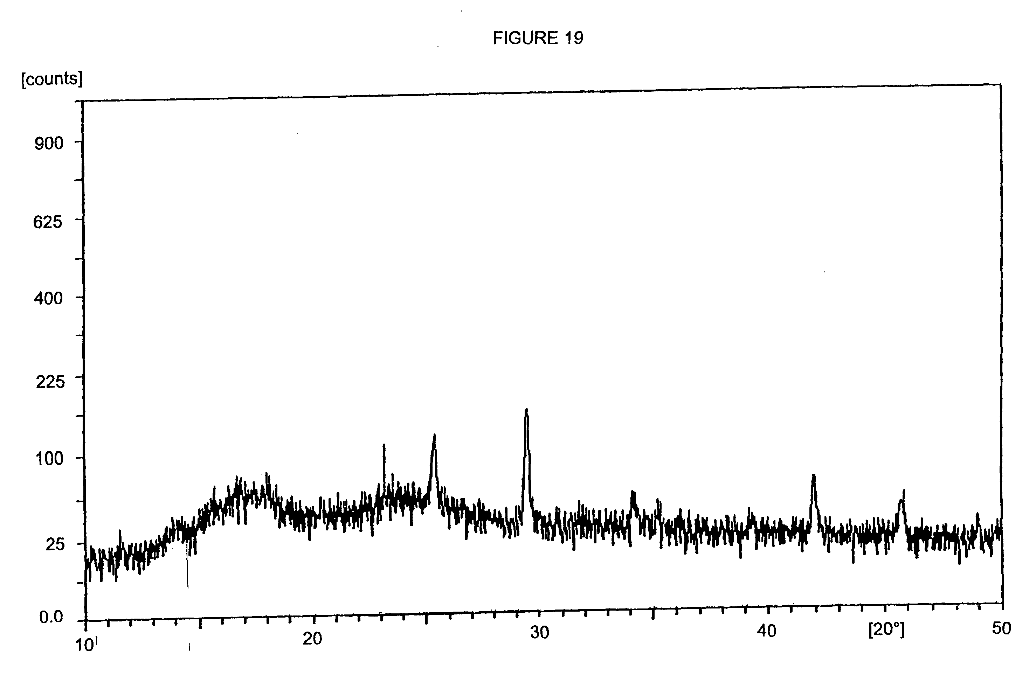

The diffraction pattern of the clathrate obtained by cogrinding according to Example 8 (Figure 19 - GRc-DHEA), is clearly different both from that of the physical mixture and from that of the clathrate obtained by precipitation (c-DHEA). An almost total disappearance of the peaks of crystallinity may be observed, and the appearance of a large undulating band typical of amorphous products. The few residual peaks of crystallinity seem to belong to the glycine. So, the X-ray analysis confirms the results of the DSC analysis, showing that the product has amorphised.

### C) INFRARED SPECTROSCOPY

The infrared spectra were obtained in Nujol, in the range 4000-600 cm¹, using an IR Perkin Elmer spectrophotometer, Mod. 983.

Due to the complex overlapping of the bands of the three components, it is not possible to obtain a great deal of information from the spectrum of the physical mixture of DHEA-α-Cyclodextrin-Glycine, in which, however, the principal DHEA absorption bands are recognisable.

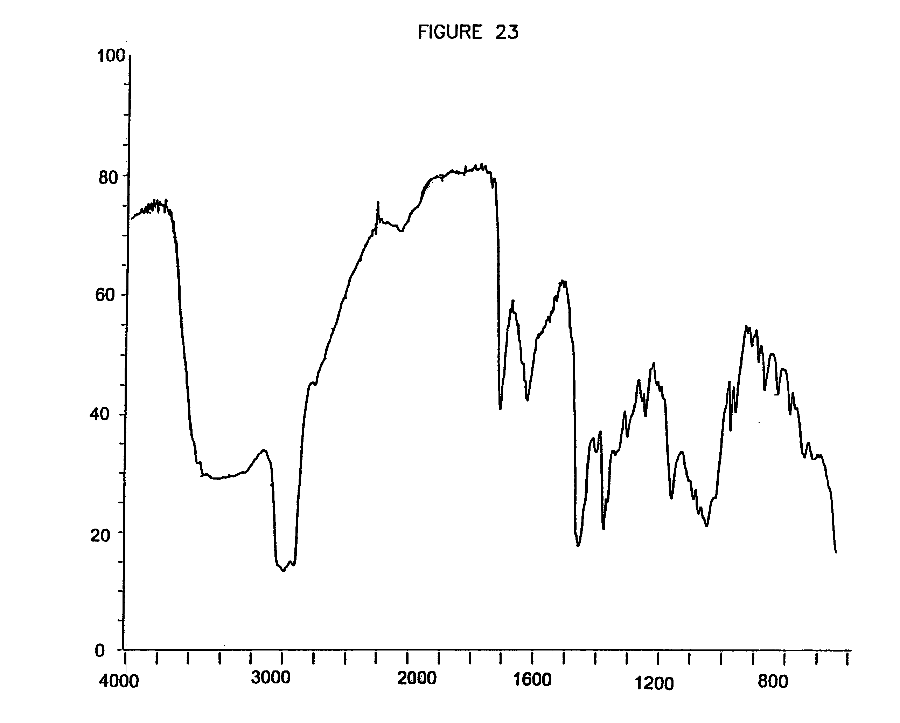

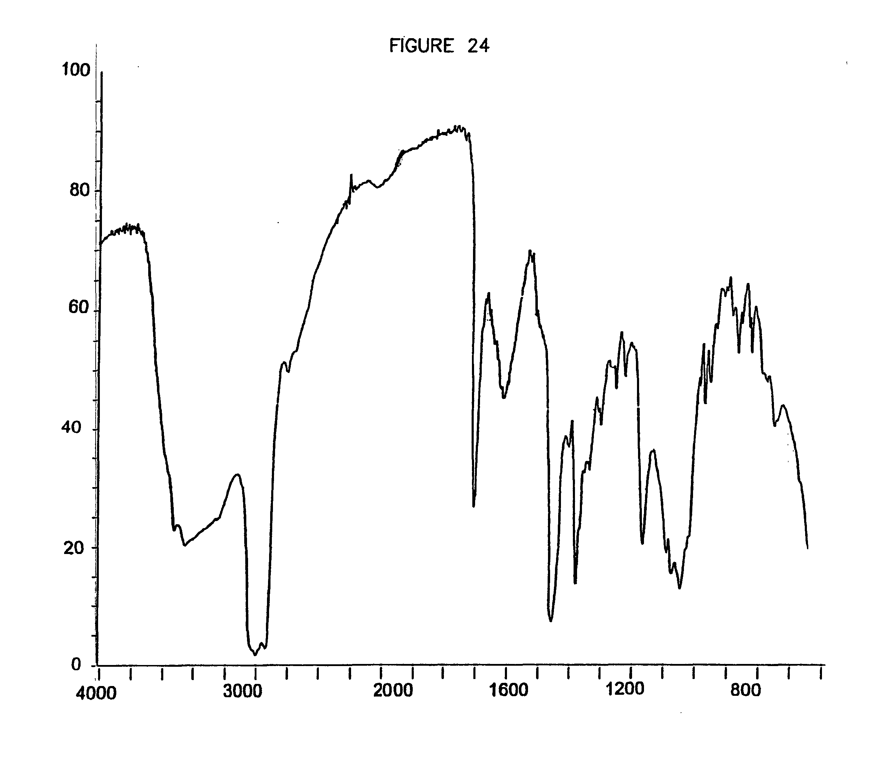

From the comparison of the IR spectrum of the physical mixture (Figure 23 - P.M.) and that of the clathrate obtained by precipitation according to Example 11 (Figure 24 - c-DHEA), the almost total correspondence of the principal bands may be noticed, for which no shifts worthy of note are observed. The most obvious difference is the increase of the relative intensity of the band at 1728 cm¹, which in the clathrate also seems to be shifted to 1724 cm¹.

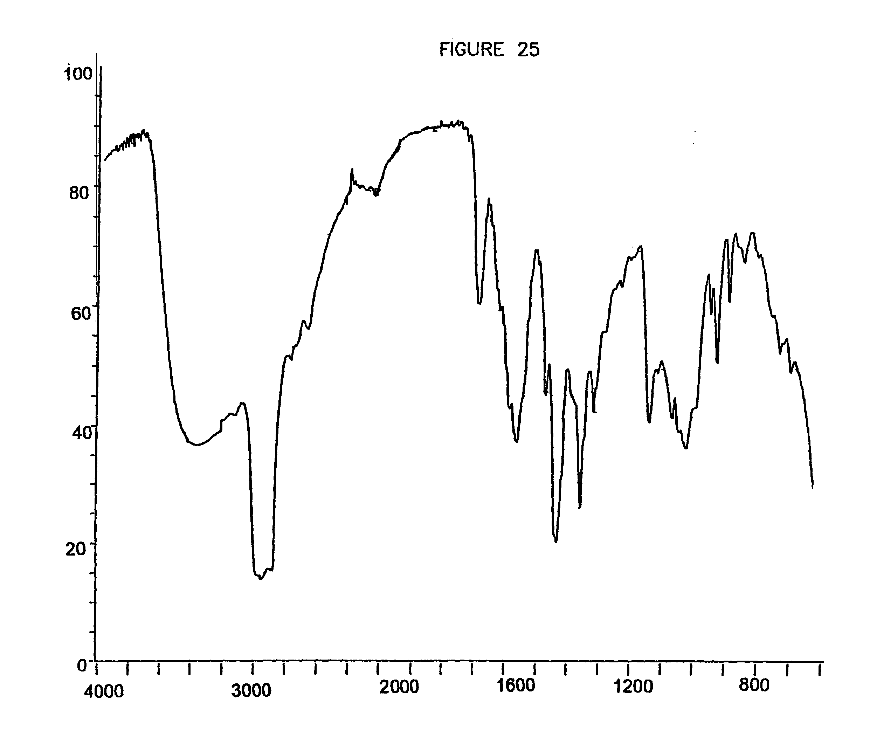

In the case of the spectrum of the clathrate obtained by cogrinding according to Example 8 (Figure 25 - GRc-DHEA), the differences with respect to the spectrum of the physical mixture are much more clearly seen. Besides a distinct decrease of the band at 1728 cm¹ (here shifted to 1731 cm⁻¹), a distinct variation is observed of the band at 1637 cm⁻¹, which increases distinctly in intensity and undergoes a shift to 1598 cm¹. The appearance of a band at 1498 cm¹ is also observed and the shift of the band at 1303 cm¹ to a higher frequency (1335 cm¹).

Though difficult to interpret specifically, due to the complexity of the spectrum of the multicomponent system, these variations indicate an intense interaction between the components, occurring after the grinding process.

### Pharmacological experimentation

### A) Kinetics and metabolism of exogenous DHEA

This first part of the experimentation was carried out with the purpose of verifying the kinetics and processes of sulfoconjugation and bioconversion of DHEA as such administered to male subjects. The said subjects were divided into two groups according to age: one group of 7 young subjects aged between 24 and 32, and one group of 10 elderly subjects, aged between 67 and 86. Each one of these subjects was administered 100 mg of DHEA in a single dose by oral route. At 0.5, 1, 2, 3, 4, 6, 9,12 and 24 hours from administration, the blood levels of DHEA, DHEA sulphate, total testosterone and 17-β-estradiol were determined. The evaluations were carried out using the parameter of the maximum delta (maximum peak reached, from which the basal values were subtracted) and the parameter of the area under the curve (triangulation system).

The results showed that:
1- The DHEA blood level peak after administration of exogenous DHEA occurred at approximately one hour from administration in both groups. Response to administration was significantly greater in the elderly subjects.
2- The DHEA sulphate blood level peak after administration of exogenous DHEA occurred at approximately two hours from administration in both groups. Response to administration was again significantly greater in the elderly subjects.
3- The molar ratio between DHEA sulphate and DHEA in the response-to-administration curves did not show any significant differences.
4- After administration of DHEA, the profile of the testosterone levels did not undergo any substantial differences in the young subjects when a comparison was made between what occurred after administration of DHEA and what occurred after administration of placebo. The difference, instead, was statistically evident in the elderly subjects (two-way ANOVA statistical analysis).
5- the same phenomenon occurred for the blood levels of 17-β-estradiol.

These results suggest the following interpretations:
a) The greater bio-availability of both DHEA and DHEA sulphate could be induced by the lower blood levels of these steroids found in basal conditions in elderly persons. A second interpretation of the phenomenon could point towards a different metabolic clearance in the two groups.
b) The sulfoconjugation process, as is suggested by the molar ratio finding, is practically identical in the two groups.
c) Bioconversion into testosterone and 17-β-estradiol is evident only in elderly persons. It would seem every likely that the basal blood level of testosterone exerts a determining role in directing peripheral bioconversion, given that this is more substantial where the blood level of testosterone is lower.

### B) Kinetics and metabolism of exogenous DHEA clathrate

This second part of the experimentation was conducted with the purpose of comparing the effects of administration of the DHEA clathrate according to the present invention with the administration of DHEA as such.

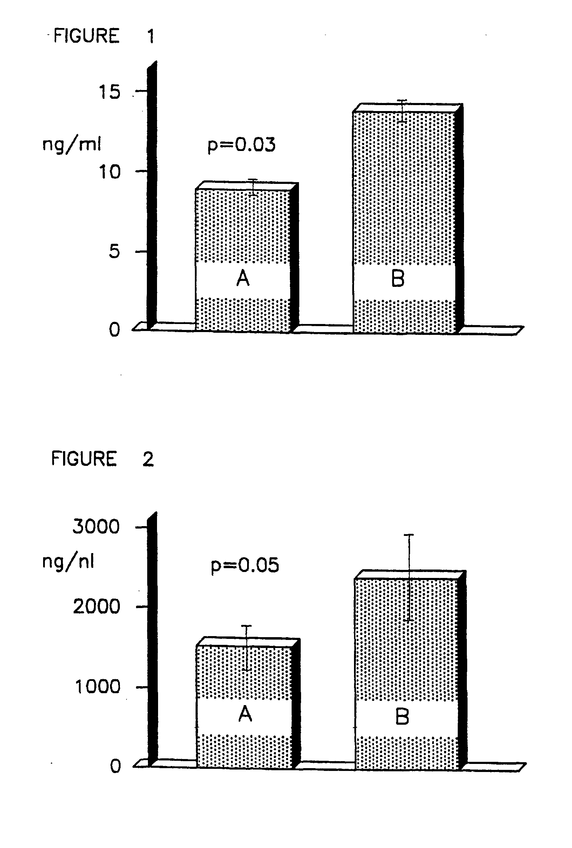

A group of 6 male subjects aged between 62 and 93 (mean age 82.3; standard deviation 11.3; standard error 4.6) were administered, in a randomized way and at 5 days apart, 25 mg of DHEA as such and DHEA in the form of clathrate as obtained from Example 4 referred to above. Determination of the blood levels of DHEA, DHEA sulphate, total testosterone, and 17-β-estradiol was carried out using the methods described in point A) above. The results obtained, which are quantified in the diagrams of Figures 1 to 7, showed that:
1- The blood levels of DHEA and DHEA sulphate were higher after administration of DHEA clathrate than after administration of DHEA as such.
2- The molar ratio between DHEA sulphate and DHEA in the blood was higher after administration of DHEA clathrate, even though the difference did not reach the limits of statistical evidence.
3- The bioconversion of DHEA into testosterone showed a behaviour that was very much the same after the two different administrations, notwithstanding the fact that the greater bio-availability of the DHEA clathrate led to clearly higher blood levels.
4- The bioconversion of DHEA into 17-β-estradiol was significantly higher after administration of DHEA clathrate than that obtained after administration of DHEA as such. This finding acquires considerable importance from the clinical standpoint if the capacity of protection of the cardiovascular system exerted by oestrogens is considered.

### Description of the figures relevant to the pharmacological experimentation

Figure 1 represents the blood levels of DHEA expressed in ng/ml after administration of DHEA as such (column A) and DHEA clathrate (column B).

The determinations were made using the maximum delta method.

Figure 2 represents the blood levels of DHEA expressed in ng/ml after administration of DHEA as such (column A) and DHEA clathrate (column B).

The determinations were made using the area-under-the-curve method.

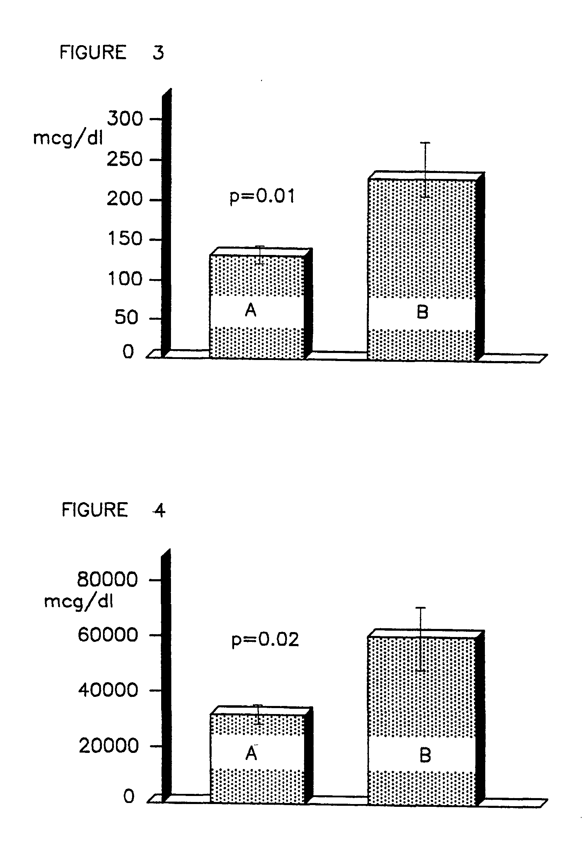

Figure 3 represents the blood levels of DHEA sulphate expressed in mcg/dl after administration of DHEA as such (column A) and DHEA clathrate (column B).

The determinations were made using the maximum delta method.

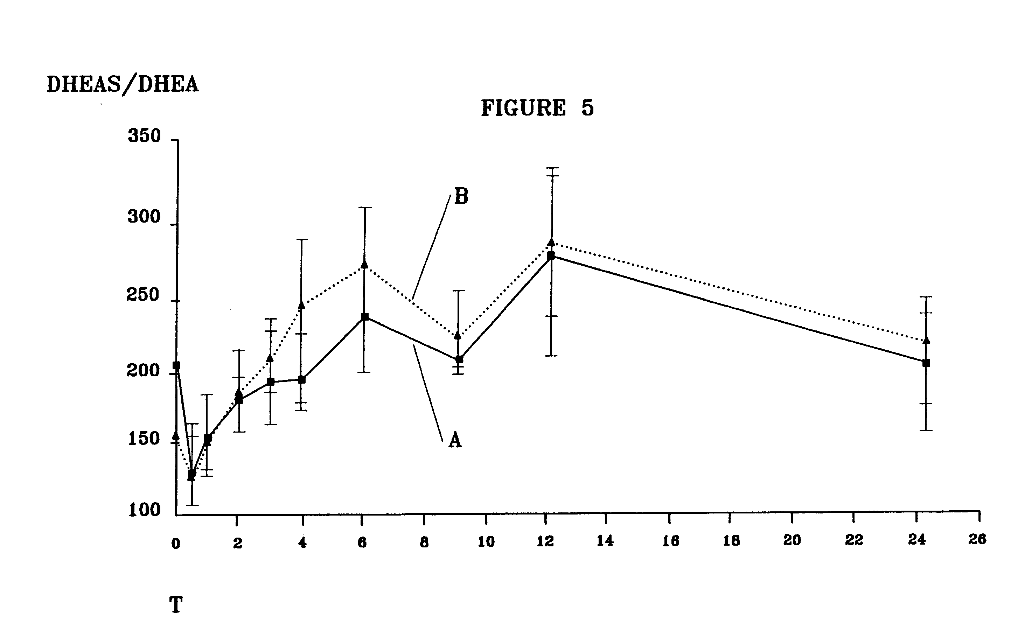

Figure 4 represents the blood levels of DHEA sulphate expressed in mcg/dl after administration of DHEA as such (column A) and DHEA clathrate (column B).

The determinations were made using the area-under-the-curve method.

Figure 5 represents the molar ratio between DHEA sulphate and DHEA in the blood as a function of time (hours) after administration of DHEA as such (curve A) and DHEA clathrate (curve B).

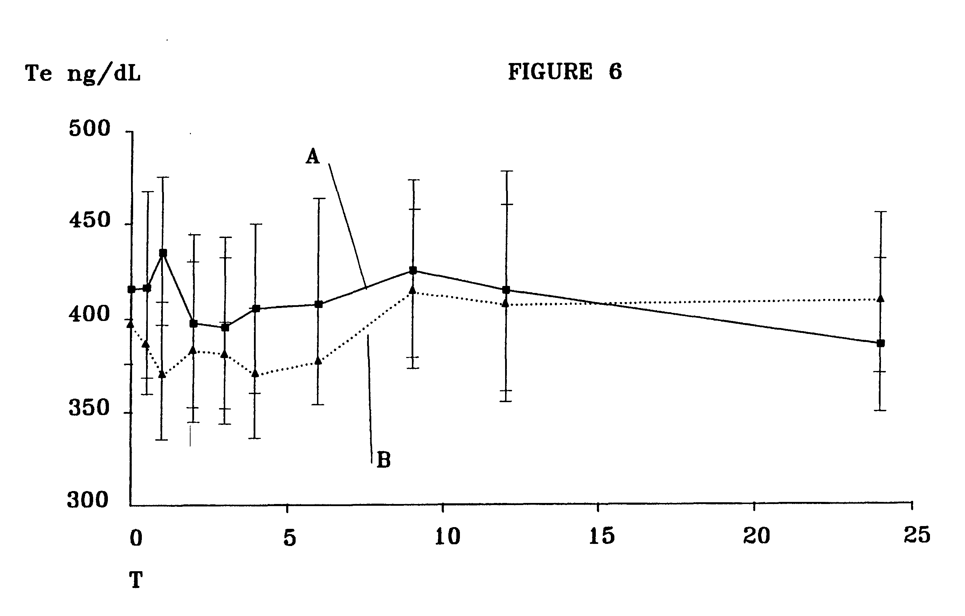

Figure 6 represents the levels of testosterone in the blood expressed in ng/dl as a function of time (hours) after administration of DHEA as such (curve A) and DHEA clathrate (curve B).

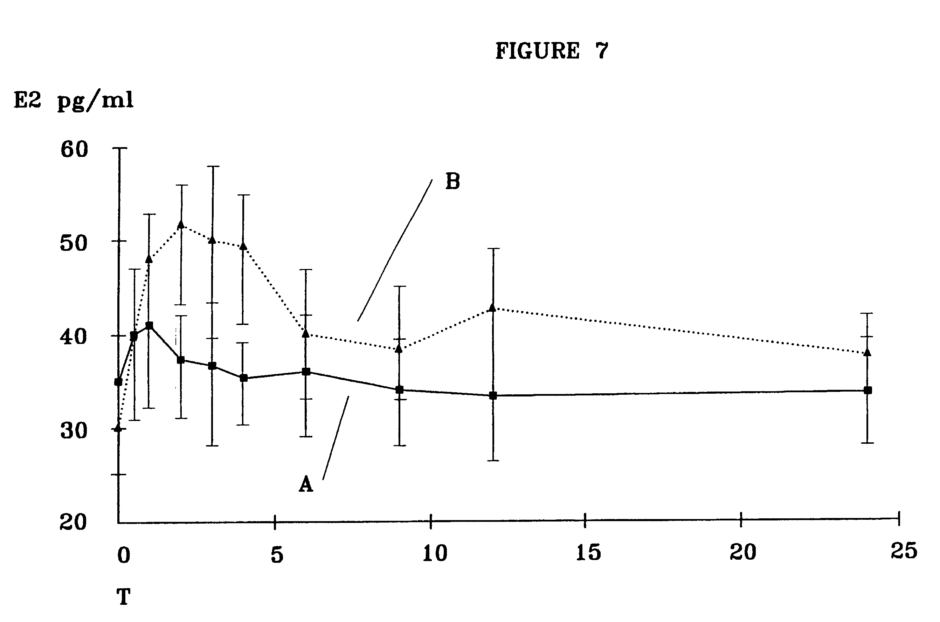

Figure 7 represents the levels of 17-β-estradiol in the blood expressed in pg/ml as a function of time (hours) after administration of DHEA as such (curve A) and DHEA clathrate (curve B).

## Claims

1. Clathrate of dehydroepiandrosterone (DHEA) possibly in the form of sulphate, in a matrix of a α-, β- or γ-cyclodextrin **characterized by** containing a co-solubilization agent selected from the group comprising an amino acid, crospovidone, polyethylene glycol 4000, cellulose, carboxymethyl cellulose, starch, tartaric acid, succinic acid, and glutaric acid.

2. Clathrate according to Claim 1, **characterized in that** said amino acid is selected from the group comprising glycine, lysine, serine and aspartate.

3. Clathrate according to Claim 1, **characterized in that** the molar ratio between said DHEA and said cyclodextrin is between 3 : 1 and 1 : 10.

4. Clathrate according to Claim 1, **characterized in that** the molar ratio between said DHEA and said cyclodextrin is 1 : 1.

5. Clathrate according to claim 1, **characterized in that** the molar ratio between said DHEA and said α-cyclodextrin is 1:1.

6. Clathrate according to Claim 1, **characterized in that** the weight amount of said co-solubilization agent is between 5 and 50%.

7. Clathrate according to Claim 1, **characterized in that** the weight amount of said co-solubilization agent is 10%.

8. Pharmaceutical compositions comprising a pharmacologically effective amount of a clathrate according to Claim 1, in a mixture with excipient and diluent substances normally used in pharmaceutical techniques.

9. Compositions according to Claim 8, **characterized in that** one part of the composition is formulated so as to enable a fast release of DHEA, and the remaining part is formulated so as to enable a slow release of DHEA.

10. Compositions according to Claim 8, suitable for the treatment of pathological conditions comprising disorders resulting from adrenocortical insufficiency, adrenopausal disorders, hypogonadism and xerophthalmia.

11. Use of clathrate according to claim 1 for the preparation of compositions for the treatment of pathological conditions comprising disorders resulting from adrenocortical insufficiency, adrenopausal disorders, hypogonadism and xerophthalmia.

12. Use according to claim 11, **characterised in that** said compositions comprise a unit dose for oral administration of from 12.5 to 50 mg of DHEA in the form of clathrate according to claim 1.

## Patentansprüche

1. Ein Clathrat von Dehydroepiandrosteron (DHEA), möglicherweise in Sulfatform, in einer Matrix aus einem α-, β- oder γ-Cyclodextrin, charakterisiert dadurch, dass ein Agens zur Co-Solubilisation enthalten ist, ausgewählt aus der Stoffgruppe, welche eine Aminosäure, Crospovidon, Polyethylenglycol 4000, Cellulose, Carboxymethylcellulose, Stärke, Weinsäure, Bemsteinsäure und Glutarsäure beinhaltet.

2. Ein Clathrat gemäß Anspruch 1, das dadurch charakterisiert ist, dass die besagte Aminosäure aus der Gruppe ausgewählt wird, die Glycin, Lysin, Serin und Aspartat umfasst.

3. Ein Clathrat gemäß Anspruch 1, das dadurch charakterisiert ist, dass das molare Verhältnis zwischen besagtem DHEA und besagtem Cyclodextrin 3:1 und 1:10 beträgt.

4. Ein Clathrat gemäß Anspruch 1, das dadurch charakterisiert ist, dass das molare Verhältnis zwischen besagtem DHEA und besagtem Cyclodextrin 1:1 beträgt.

5. Ein Clathrat gemäß Anspruch 1, das dadurch charakterisiert ist, dass das molare Verhältnis zwischen besagtem DHEA und besagtem α-Cyclodextrin 1:1 beträgt

6. Ein Clathrat gemäß Anspruch 1, das dadurch charakterisiert ist, dass der Gewichtsanteil des besagten Agens für die Co-Solubilisation zwischen 5 und 50 % liegt.

7. Ein Clathrat gemäß Anspruch 1, das dadurch charakterisiert ist, dass der Gewichtsanteil des besagten Agens für die Co-Solubilisation bei 10% liegt.

8. Pharmazeutische Zubereitungen, die eine pharmakologisch wirksame Menge an Clathrat gemäß Anspruch 1 enthalten, in einer Mischung mit Träger- und Verdünnungssubstanzen, die üblicherweise in der pharmazeutischen Technik eingesetzt werden.

9. Zubereitungen gemäß Anspruch 8, die dadurch charakterisiert sind, dass jeweils ein Teil der Zubereitung in einer Formulierung vorliegt, die eine rasche Freisetzung und der andere Teil in einer Formulierung besteht, die eine langsame Freisetzung von DHEA ermöglicht.

10. Zubereitungen gemäß Anspruch 8, die für die Therapie pathologischer Zustände geeignet sind, zu denen Erkrankungen zählen, die durch Nebennierenrindeninsuffizienz, Störungen der Adrenopause, Hypogonadismus und Xerophthalmie hervorgerufen werden.

11. Der Einsatz von Clathrat gemäß Anspruch 1 zur Herstellung der Zubereitungen für die Therapie pathologischer Zustände, zu denen solche Erkrankungen zählen, die durch Nebennierenrindeninsuffizienz, Störungen der Adrenopause, Hypogonadismus und Xerophthalmie hervorgerufen werden.

12. Der Einsatz gemäß Anspruch 11, der dadurch charakterisiert ist, dass die besagten Zubereitungen eine Einheitendosierung für die orale Verabreichung von 12,5 bis 50 mg an DHEA in Form von einem Clathrat gemäß Anspruch 1 enthalten.

## Revendications

1. Clathrate de déhydroépiandrostérone (DHEA) éventuellement sous forme de sulfate, dans une matrice d' α-, β- ou γ-cyclodextrine **caractérisé en ce qu'**il contient un agent de co-solubilisation choisi dans le groupe comprenant un acide aminé, de la crospovidone, du polyéthylène glycol 4000, de la cellulose, de la carboxyméthyle-cellulose, de l'amidon, de l'acide tartrique, de l'acide succinique et de l'acide glutarique.

2. Clathrate selon la revendication 1, **caractérisé en ce que** ledit acide aminé est choisi dans le groupe comprenant la glycine, la lysine, la sérine et l'aspartate.

3. Clathrate selon la revendication 1, **caractérisé en ce que** le rapport molaire entre ladite DHEA et ladite cyclodextrine est compris entre 3 : 1 et 1 : 10.

4. Clathrate selon la revendication 1, **caractérisé en ce que** le rapport molaire entre ladite DHEA et ladite cyclodextrine est de 1 : 1.

5. Clathrate selon la revendication 1, **caractérisé en ce que** le rapport molaire entre ladite DHEA et ladite α-cyclodextrine est de 1 : 1.

6. Clathrate selon la revendication 1, **caractérisé en ce que** la quantité de poids dudit agent de co-solubilisation est comprise entre 5 et 50 %.

7. Clathrate selon la revendication 1, **caractérisé en ce que** la quantité de poids dudit agent de co-solubilisation est de 10 %.

8. Compositions pharmaceutiques comprenant une quantité efficace sur le plan pharmacologique d'un clathrate selon la revendication 1, dans un mélange avec un excipient et des diluants normalement utilisés dans les techniques pharmaceutiques.

9. Compositions selon la revendication 8, **caractérisées en ce qu'**une partie de la composition est formulée de manière à permettre une libération rapide de DHEA et l'autre partie est formulée de manière à permettre une libération lente de DHEA.

10. Compositions selon la revendication 8, appropriées au traitement de conditions pathologiques comprenant des troubles résultant d'une insuffisance corticosurrénale, de l'adrénopause, d'un hypogonadisme ou d'une xérophtalmie.

11. Utilisation de clathrate selon la revendication 1 pour la préparation de compositions pour le traitement de conditions pathologiques comprenant des troubles résultant d'une insuffisance corticosurrénale, de l'adrénopause, d'un hypogonadisme ou d'une xérophtalmie.

12. Utilisation selon la revendication 11, **caractérisée en ce que** lesdites compositions comprennent une dose unitaire pour administration orale de 12,5 à 50 mg de DHEA sous forme de clathrate selon la revendication 1.
